# EUROPEAN PATENT APPLICATION

(11) **EP 4 607 196 A2**
(43) Date of publication of application: **27.08.2025**
(21) Application number: 25159475.0
(22) Date of filing: 21.02.2025
(51) Int. Cl.: G01N 33/543, A61B 5/145, G16B 40/00

(54) **INSILICO METHOD AND SYSTEM FOR DESIGNING A BASELINE PEPTIDE BIORECEPTOR FOR SENSING A BIOMARKER FOR DYSGLYCEMIC DISORDERS**

(30) Priority: 23.02.2024 IN 202421013153
(71) Applicant: Tata Consultancy Services Limited, Maharashtra (IN)
(72) Inventor: DESHPANDE, Parijat Dilip, 411028 Pune, Maharashtra (IN); DE, Debankita, 560066 Bangalore (IN); RAI, Beena, 411028 Pune. Maharashtra (IN)
(74) Representative: Goddar, Heinz J.

(57) **Abstract**

This disclosure relates generally to a method and system for designing a baseline peptide bioreceptor. State-of-the-art methods provide peptide designing through specific target selection and through desired conformational stability. However, considering individual properties of amino acid while designing a peptide sequence have a greater role in imparting stability in designing the peptide sequence. The disclosed method provides a baseline peptide sequence by identifying active binding sites for a ligand using a computational docking technique. The active sites are selected based on binding affinity of protein-ligand complex. Further, selected binding sites are utilized in identifying energetically favorable interactions of protein-ligand complex through molecular dynamics simulation performed in a biofluid environment. Finally, multi-parameter optimization model with parameters such as sequence length, binding affinity etc. is executed to obtain the baseline peptide.

## Description

### CROSS-REFERENCE TO RELATED APPLICATIONS AND PRIORITY

The present application claims priority from Indian patent application no. 202421013153, filed on Feb 23, 2024.

### TECHNICAL FIELD

The disclosure herein generally relates to a biosensor for glucose monitoring, and, more particularly, to a peptide-based biosensor with a wearable glucose monitoring device.

### BACKGROUND

Diabetes is a group of metabolic diseases characterized by hyperglycemia, and there is currently no cure for diabetes. The treatment of diabetes is mainly to monitor and control blood sugar levels. The traditional method of blood glucose measurement has obvious defects, which brings trauma and pain to the patient during the measurement process, and it is not convenient to achieve continuity detection. Non-invasive blood glucose detection technology overcomes the shortcomings of traditional detection methods and can effectively meet the needs of diabetics to monitor blood glucose concentration in real time and frequently.

Various non-invasive techniques to monitor glucose level exist which are far more convenient than the invasive methods. Wrist-mountable devices having an electrode measures glucose present in sweat at the skin surface. Infrared based glucose sensor mountable are available that can be mounted on a wrist or finger. Device that estimates blood glucose through illuminating a patient's eye with near- infrared radiation. Further, devices measuring blood glucose within blood vessels of a tympanic membrane in a human ear through light absorption measurements. Non-invasive blood glucose detection methods are mainly concentrated in the field of optical detection. Due to many interference components and large individual differences, most of the detection methods are still in the laboratory research stage.

Proteins represent a promising class of biorecognition elements that can be coupled to electrochemical transducers. The proteins are generally used to bind to biomarkers such as glucose for tracing or determining various disorders in organisms. Biomarkers can be tracked in samples with various origins and in different concentrations, revealing whether an organism is in a healthy or unhealthy state. In regard to detection, electrochemical biosensors are a potential fusion of electronics, chemistry, and biology, allowing for fast and early point-of-care detection from a biological sample with the advantages of high sensitivity, simple construction, and easy operation. However, the detection of specific biomolecules is usually challenging because of the low concentration in samples and the possibility of nonspecific interactions due to interfering species in the biofluid matrix.

Measuring an analyte of interest through non-invasive means using protein-based biosensors becomes even more challenging when the analyte is to be collected from biofluids, such as sweat or saliva. These biofluids have a very low quantity of analyte concentration to be detected. The levels of glucose in sweat lie in the range of 0.06 to 0.2 millimolar (mM). The normal glucose levels in saliva are 0.5-1.00 mg/100 ml. Detecting such small quantities through protein biosensors require a peptide structure that can effectively be immobilized onto the surface of electrode coated with a gold substrate for subsequent transduction of signals. Antibody based electrochemical sensors have been traditionally used for the measurement of glucose and other biomolecules due to their high specificity and sensitivity. However, their limitations such as storage requirement, temperature instability, high cost, cross-reactivity, and batch-to-batch variability have prompted us to explore further. Antibodies can be prone to denaturation and degradation, which can affect their binding affinity and specificity.

### SUMMARY

Embodiments of the present disclosure present technological improvements as solutions to one or more of the above-mentioned technical problems recognized by the inventors in conventional systems. For example, in one aspect, a baseline peptide bioreceptor is provided. The baseline peptide bioreceptor comprising an amino acid sequence of the general formula:

N-terminus-CYS-W-X-C-terminus;

wherein X is a sequence of 1-5 charged amino acid;
W is a sequence of between 2 and 70 amino acids of any type, wherein more than 50% of the total amino acids represented by W, are non-polar amino acid; and
wherein the peptide binds to the gold substrate from N-terminus side utilizing thiol bond of cysteine (CYS) amino acid.
The baseline peptide bioreceptor is optimized having a binding energy of range of about 3.0Kcal/J to -7.5Kcal/J for a ligand-bioreceptor complex. The baseline peptide bioreceptor designed according to present disclosure is represented as SEQ ID No 1.

In another aspect, a method for designing a baseline peptide bioreceptor for sensing a biomarker of dysglycemic disorders is provided. The method includes obtaining, via one or more hardware processors, protein files of a plurality of proteins expressed in dysglycemic disorders from a public repository. The plurality of files of naturally derived protein were imported from protein data bank (PDB), a public repository. The naturally available proteins like glucose/ galactose binding protein (GGBP), sugar binding proteins, transport proteins, lectins, etc were imported. The method further includes pre-processing of the plurality of proteins, via the one or more hardware processors. The pre-processing involves lot many curations to offer a protein before it can be docked. All the non-protein parts are identified and removed from the protein, such as ligands (HETATM), ions (CL, NA etc) and water (SOL). Then, the missing hydrogens are added. Further, appropriate force fields were selected, Kollman charges were added. The pre-processed files are stored internally as PDBQT files compatible with the AutoDock Vina^{™}, a protein docking software. Similarly, ligand is also pre-processed to ensure completeness of the ligand. Generally, the ligand lacks hydrogen atoms, so hydrogen atoms are added to the ligand and rotatable bonds are defined that are used for flexible docking. The pre-processing of proteins and ligands are executed in AutoDock Vina^{™}. The method further includes docking the plurality of proteins with a ligand to obtain a plurality of protein-ligand complex, wherein the plurality of protein-ligand complex is ranked based on predicted binding affinities. For each protein-ligand complex being assessed by the docking study, the protein is placed in a grid box of known dimensions, the ligand is placed in its vicinity, and the binding pose, and binding energy are obtained by optimizing the potential energy. The method further includes simulating the plurality of ranked protein-ligand complex in a biofluid model to obtain energetically favorable protein-ligand interactions. Based on docking, the most favorable interactions between protein and ligand is identified based on their binding affinity. From the stability and specificity purpose, the spatial arrangement of the residues is performed using molecular dynamics (MD) simulation. The MD simulation performed umbrella sampling using GROMACS^{™} to explore and calculate the free energy profile of a specific reaction coordinate for understanding the energy landscape of the process. The method further includes optimizing a plurality of stability parameters by docking the stable protein-ligand complex. The plurality of parameters comprises selectivity, pH, solubility, sequence length and sequence structure. The effect of all these parameters on the protein-ligand interaction are analyzed individually as well as in combination of more than one parameter at a time to identify deviations in behavior of the interaction. The method further includes identifying the peptide sequence of the protein involved in the protein-ligand complex. The active binding sites of the top candidate proteins which are found to be most favorable, energetically, were analyzed and candidate continuous sequence of amino acids (< 40 AA) amongst these candidates were iteratively assessed to arrive at a baseline peptide bioreceptor retaining its existing scaffold. Parameters such as binding energy, sequence length, tertiary structure, cost of synthesis, solubility, pH etc. were considered to arrive at the presented peptide. The peptide is modelled in AutoDock Vina^{™} to establish comparable binding energy values and suitable peptide structure. The candidate peptides are optimized by iteratively selecting various continuous lengths from active binding sites of top proteins to arrive at the proposed baseline peptide. The method further includes replacing N-terminus amino acid of the peptide sequence with cysteine amino acid to obtain cysteine modified peptide sequence. The N-terminal is selected as cysteine existing from the native protein which serves as a thiol bond with the gold electrode substrates for further development as a biosensor. The method further includes performing simulation of the cysteine modified peptide sequence in a biofluid model by computing root mean square deviation (RMSD) to obtain the baseline peptide bioreceptor. The cysteine modified peptide sequence is taken for simulation in a biofluid model by computing RMSD to obtain the baseline peptide. The simulation is performed in GROMACS^{™} for 10ns to ensure stability. The peptide modelled using GROMACS^{™} to assess its root mean square deviation (RMSD) and binding energetics via umbrella sampling and computing PMF and compared with native protein which serves as a benchmark. The approach of designing baseline peptide is biomimetically inspired i.e., selecting a continuous sequence of amino acids from the native protein, correspondingly retaining structure, scaffold and stability of the finalized baseline peptide as opposed to combinatorial peptide design. The baseline peptide bioreceptor so designed detects a biomarker in a test sample by a process comprising steps: contacting the test sample with a surface of the biosensor; permitting signal generation to occur as the biomarker contacts the baseline peptide of the biosensor; and detecting the presence or amount of the biomarker in the test sample using a detection assembly.

In another aspect, a biosensor for sensing the presence of analyte in the biofluid of the human is provided. The biofluid is selected from one or more sweat, saliva, urine, blood and the like. The biosensor comprises of a baseline peptide bioreceptor; and a transducer e.g. electrochemical, SPR etc, wherein the baseline peptide binds with the biomarker ligand and a detectable signal is transduced through cysteine amino acid attached to a gold electrode of the transducer, and wherein a signal transduced by the baseline peptide bound to the biomarker differs from a signal transduced by the baseline peptide when the baseline peptide is not bound to the biomarker, and wherein the baseline peptide comprises of more than 50% non-polar amino acid. The baseline peptide designed according to the present disclosure is in the form of a kit comprising a biosensor comprising a baseline peptide; a unit for signal processing; and a means for wireless transmission.

In another aspect, a wearable device for monitoring of biomarkers of dysglycemic disorders is provided. The wearable device comprises: a biosensor array comprising a baseline peptide along a circumference of the wearable device including at least one electromagnetic energy emitter and at least one electromagnetic energy receiver; a power source; a data processor which receives data from the electromagnetic energy receiver which is analyzed in order to measure glucose level of a subject; and a data transmitter.

In another aspect, a system for designing a baseline peptide bioreceptor for sensing a biomarker of dysglycemic disorders is provided. The system includes at least one memory storing programmed instructions; one or more Input /Output (I/O) interfaces; and one or more hardware processors, a day-ahead planning module and an intra-day planning module, operatively coupled to a corresponding at least one memory, wherein the system is configured to obtain, via one or more hardware processors, protein files of a plurality of proteins expressed in dysglycemic disorders from a public repository. Further, the system is configured to pre-process, via the more or more hardware processors, the plurality of proteins. Further, the system is configured to dock, via the more or more hardware processors, the plurality of proteins with a ligand to obtain a plurality of protein-ligand complex, wherein the plurality of protein-ligand complex is ranked based on predicted binding affinities. Further, the system is configured to simulate, via the more or more hardware processors, the plurality of ranked protein-ligand complex in a biofluid model to obtain energetically favorable protein-ligand interactions. Further, the system is configured to optimize, via the more or more hardware processors, a plurality of stability parameters by docking the stable protein-ligand complex. Further, the system is configured to identify, via the more or more hardware processors, the peptide sequence of the protein involved in the protein-ligand complex. Further, the system is configured to replace, via the more or more hardware processors, N-terminus amino acid of the peptide sequence with cysteine amino acid to obtain cysteine modified peptide sequence. Further, the system is configured to perform, via the more or more hardware processors, simulation of the cysteine modified peptide sequence in a biofluid model by computing root mean square deviation (RMSD) to obtain the baseline peptide bioreceptor.

In yet another aspect, a computer program product including a non-transitory computer-readable medium having embodied therein a computer program for designing a baseline peptide bioreceptor for sensing a biomarker of dysglycemic disorders is provided. The computer readable program, when executed on a computing device, causes the computing device to obtain, protein files of a plurality of proteins expressed in dysglycemic disorders from a public repository. Further, the computer readable program, when executed on a computing device, causes the computing device to pre-process the plurality of proteins. The computer readable program, when executed on a computing device, causes the computing device to dock, via the more or more hardware processors, the plurality of proteins with a ligand to obtain a plurality of protein-ligand complex, wherein the plurality of protein-ligand complex is ranked based on predicted binding affinities. The computer readable program, when executed on a computing device, causes the computing device to simulate the plurality of ranked protein-ligand complex in a biofluid model to obtain energetically favorable protein-ligand interactions. The computer readable program, when executed on a computing device, causes the computing device to optimize a plurality of stability parameters by docking the stable protein-ligand complex. The computer readable program, when executed on a computing device, causes the computing device to identify the peptide sequence of the protein involved in the protein-ligand complex. The computer readable program, when executed on a computing device, causes the computing device to replace N-terminus amino acid of the peptide sequence with cysteine amino acid to obtain cysteine modified peptide sequence. The computer readable program, when executed on a computing device, causes the computing device to perform simulation of the cysteine modified peptide sequence in a biofluid model by computing root mean square deviation (RMSD) to obtain the baseline peptide bioreceptor.

It is to be understood that both the foregoing general description and the following detailed description are exemplary and explanatory only and are not restrictive of the invention, as claimed.

### BRIEF DESCRIPTION OF THE DRAWINGS

The accompanying drawings, which are incorporated in and constitute a part of this disclosure, illustrate exemplary embodiments and, together with the description, serve to explain the disclosed principles:
FIG. 1 illustrates a schematic block diagram of a system for designing a baseline peptide for sensing a biomarker for dysglycemic disorders according to some embodiments of the present disclosure.
FIG. 2 illustrates sequential approach adopted by the system 100 in attempting bioreceptor design according to some of the embodiments of the present invention.
FIGS. 3A and 3B are an exemplary flow diagrams for a method for designing a baseline peptide for sensing a biomarker for dysglycemic disorders according to some embodiments of the present disclosure.
FIG. 4 illustrates molecular interaction of human hexokinase with glucose molecule according to some embodiments of the present disclosure.
FIG. 5 depicts optimization of protein-ligand complex using molecular dynamics under the influence of solvent according to some embodiments of the present disclosure.
FIG. 6 depicts potential mean force (PMF) experienced by the protein-ligand complex during molecular dynamics according to some embodiments of the present disclosure.
FIG. 7 is a plot of root mean square deviation (RMSD) depicting stability of the optimized protein structure according to some embodiments of the present disclosure.
FIG. 8 represents a bioreceptor interacting with β-D-glucose forming a protein-ligand complex with favorable energy profile according to some embodiments of the present disclosure.
FIG. 9 illustrate a baseline peptide biosensor, according to some embodiments of the present disclosure.
FIG. 10 illustrate a baseline peptide-based device sensing a biomarker in a test sample, according to some embodiments of the present disclosure.

### DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.

The term "peptide biosensor", as used herein, relates to a peptide-based bioreceptor that allows detection of the presence and/or amount, preferably both, of an analyte.

The term "peptide", as used in this context, i.e. in connection with the peptide biosensor, relates to a polymer of amino acids that are linked by peptide bonds, with said peptide having a length that is sufficient to provide for a protease recognition site and an analyte binding site. Typically, the peptide biosensors comprise at least 10-15, preferably at least 25 amino acids. Depending on the length of the peptide, the peptide may be considered a polypeptide, with such polypeptides typically having a length of 50 or more amino acids.

The term "amino acid" refers to naturally occurring and artificially produced amino acids, and also amino acid analogs and amino acid mimetics that function in a similar manner to the naturally occurring amino acids. Amino acid analogs refer to compounds that have the same basic chemical structure as the naturally occurring amino acids. It is preferred that in the peptides of the present invention, the 20 naturally occurring amino acids glycine, alanine, valine, leucine, isoleucine, phenylalanine, proline, cysteine, methionine, tyrosine, tryptophan, glutamine, asparagine, serine, threonine, glutamic acid, aspartic acid, histidine, lysine and arginine are used.

The term "docking" as used herein, refers to the computational process for simulating and/or characterizing the binding of a computational representation of a molecule (e.g., a substrate or ligand) to a computational representation of an active site of a biomolecule (e.g., an enzyme or protein). Docking is typically implemented in a computer system using a "Autodock Vina^{™}" computer program. Typically, the result of a docking process is a computational representation of the molecule "docked" in the active site in a specific "pose." A plurality of docking processes may be carried out between the same computational representation of a molecule and the same computational representation of an active site resulting in a plurality of different "poses" of the molecule in the active site. The evaluation of the structure, conformation, and energetics of the plurality of different "poses" in the computational representation of the active site can identify certain "poses" as more energetically favorable for binding between the ligand and the biomolecule.

A "ligand" is a biomolecule such as glucose or complex that interacts with an active site of a bioreceptor to form a stable complex containing at least the ligand and bioreceptor. In addition to the ligand and bioreceptor, the stable complex may include (sometimes require) other chemical entities such as organic and inorganic cofactors (e.g., coenzymes and prosthetic groups), metal ions, and the like. Ligands may be agonists or antagonists.

The "active site" of a bioreceptor is a site defined by the structure of the bioreceptor which is capable of containing and/or binding all or part of a molecule (e.g., a substrate or ligand). Many types of active sites are contemplated and some of these are described elsewhere herein. Often the active site contains chemical and/or physical features (e.g., amino acid residues) capable of forming binding interactions with the ligand. In some embodiments (e.g., when the bioreceptor is an enzyme), the "active site" includes at least one catalytic residue and a plurality of binding residues, and sometimes other chemical entities such as organic and inorganic cofactors (e.g., coenzymes and prosthetic groups), metal ions, and the like.

The terms "biopeptide" "polypeptide" and "peptide" are used interchangeably to denote a polymer of at least two amino acids covalently linked by an amide bond, regardless of length or post-translational modification (e.g., glycosylation, phosphorylation, lipidation, myristilation, ubiquitination, etc.). **In** some cases, the polymer has at least about 30 amino acid residues, and usually at least about 50 amino acid residues. The terms include compositions conventionally considered to be fragments of full-length proteins or peptides. Included within this definition are D- and L-amino acids, and mixtures of D- and L-amino acids. The polypeptides described herein are not restricted to the genetically encoded amino acids. Indeed, in addition to genetically encoded amino acids, the polypeptides described herein may be made up of, either in whole or in part, naturally-occurring and/or synthetic non-encoded amino acids. In some embodiments, a polypeptide is a portion of the full-length ancestral or parental polypeptide, containing amino acid additions or deletions (e.g., gaps) and/or substitutions, as compared to the amino acid sequence of the full-length parental polypeptide, while still retaining functional activity (e.g., catalytic activity).

The term 'sequence' is used herein to refer to the order and identity of any biological sequences including but not limited to protein, peptide, polypeptide, polysaccharide, etc. In some contexts, a "sequence" refers to the order and identity of amino acid residues in a protein (i.e., a protein sequence or protein character string).

A "peptide sequence" refers to the order and identity of the amino acids comprising a protein or peptide.

As used herein the terms "receptor", "bioreceptor", "biopeptide" are used interchangeably and refer to a peptide sequence designed as per the method disclosed in the present disclosure.

As used herein the terms "ligand", "biomarker", "analyte" are used interchangeably and refer to a chemical compound to be detected in the test sample using the biosensor designed as per the method disclosed in the present disclosure.

Glucose is a particularly important biomarker, the levels of which in various biofluids is indicative of the health and fitness of an individual. Clinical Blood Glucose tests conducted today are invasive and painful. Moreover, there is no continuous monitoring. Due to these pitfalls, the research community has turned to other biofluids. Among these, sweat is the most popular due to its inherent non-invasiveness and ease of access. It has been established that sweat glucose has a limited correlation to blood glucose levels to some extent. The levels of glucose in sweat lie in the range of 0.06 to 0.2 mM. Wearable devices are commercially available today that help monitor fitness parameters like heart rate, blood oxygen levels, sleep times, etc. But there is increasing demand for wearables that can monitor biomarker levels as well. The present disclosure demonstrates synthetic receptors for such glucose sensors. For the choice of a bioreceptor, various naturally available proteins like glucose/ galactose binding protein (GGBP), sugar binding proteins, transport proteins, lectins, etc are considered. The interaction of the native protein with glucose is studied using AutoDock Vina^{™}. The binding energy of the docked complex is used to rank receptors for their suitability for sensor design.

Commercially available wearable devices can monitor fitness parameters like heart rate, blood oxygen levels, etc. In comparison to that, wearable biosensors based on metabolite monitoring can provide a more detailed and in-depth picture of the health and well-being of an individual at a molecular level. A biochemical assessment of sweat can provide insights into dehydration, substance abuse, stress, and disease advancement. Sweat glucose levels have been correlated to blood glucose levels to some extent and it may serve as a basis for further tests for diagnosis of diabetes. Recently, many subcutaneously implantable glucose sensors, sensors for continuous glucose monitoring (CGM), sweat and ISF based biosensors have been reported. Currently, proteins sensitive to glucose (referred to as glucose-sensitive proteins, in literature) such as Glucose Oxidase, hexokinase, glucokinase, concanavalin A (Con-A), are used in commercial glucose sensors. It is thus vital to synthesize glucose-sensitive receptors which are biocompatible, non-toxic, independent of environmental factors such as ions, presence of monovalent/ divalent ions, etc., and cost-effective. The present disclosure is about the development of an inexpensive, synthetic peptide that can be considered as an alternative to the antibodies.

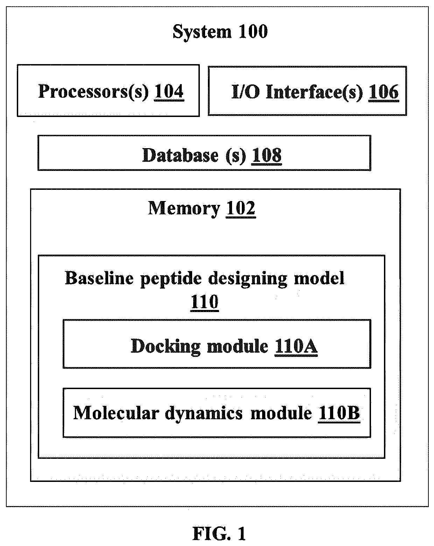

Referring now to the drawings, and more particularly to FIG. 1 through FIG. 10, where similar reference characters denote corresponding features consistently throughout the figures, there are shown preferred embodiments, and these embodiments are described in the context of the following exemplary system and/or method.

FIG. 1 illustrates a schematic block diagram of a system for designing a peptide for detecting or monitoring a biomarker for dysglycemic disorders according to some embodiments of the present disclosure.

As illustrated in FIG. 1, the system 100 designs a biomimetic bioreceptor, precisely, a peptide, to identify a suitable candidate having an affinity and a favorable interaction with the biomarker to be detected i.e glucose. The system 100 utilizes docking and molecular dynamics tools to design a baseline peptide capable of binding β-D-Glucose, a biomarker for monitoring dysglycemic disorders such as diabetes. It should be appreciated that analysis using any other model is well within the scope of this disclosure. Similarly, identification of other biomarkers such as cortisol, progesterone are well within the scope of this disclosure.

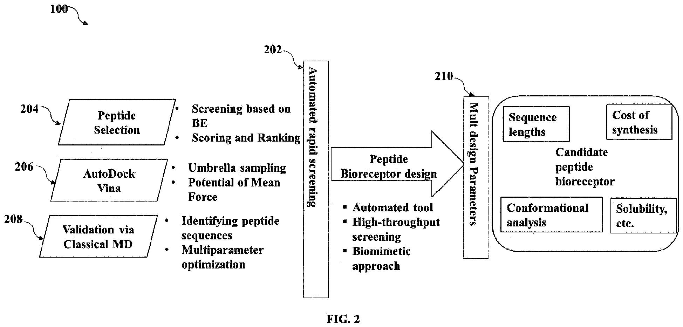

In an embodiment, the system 100 includes one or more processors 104, communication interface device(s) or input/output (I/O) interface(s) 106, and one or more data storage devices or memory 102 operatively coupled to the one or more processors 104. The one or more processors 104 that are hardware processors can be implemented as one or more microprocessors, microcomputers, microcontrollers, digital signal processors, central processing units, state machines, graphics controllers, logic circuitries, and/or any devices that manipulate signals based on operational instructions. Among other capabilities, the processor(s) are configured to fetch and execute computer-readable instructions stored in the memory. In the context of the present disclosure, the expressions 'processors' and 'hardware processors' may be used interchangeably. In an embodiment, the system 100 can be implemented in a variety of mobile computing systems, such as mobile devices, laptop computers, notebooks, hand-held devices, workstations, mainframe computers, servers, a network cloud and the like. The I/O interface (s) 106 may include a variety of software and hardware interfaces, for example, a web interface, a graphical user interface, and the like and can facilitate multiple communications within a wide variety of networks and protocol types, including wired networks, for example, LAN, cable, etc., and wireless networks, such as WLAN, cellular, or satellite. In an embodiment, the I/O interface(s) 106 can include one or more ports for connecting a number of devices to one another or to another server. The memory 104 may include any computer-readable medium known in the art including, for example, volatile memory, such as static random-access memory (SRAM) and dynamic random-access memory (DRAM), and/or non-volatile memory, such as read only memory (ROM), erasable programmable ROM, flash memories, hard disks, optical disks, and magnetic tapes. In an embodiment, the memory 102 may include a database or repository. The memory 102 may comprise information pertaining to input(s)/output(s) of each step performed by the processor(s) 104 of the system 100 and methods of the present disclosure. In an embodiment, the database may be external (not shown) to the system 100 and coupled via the I/O interface 106. The memory 102, further include baseline peptide designing model 110. The baseline peptide designing model 110 computationally, builds the baseline peptide by utilizing docking module 110A and molecular dynamics module 110B. The baseline peptide designing model 110 integrates the molecular docking using python scripting in AutoDock Vina^{™} and molecular dynamics simulations using GROMACS^{™} and design baseline candidate peptide bioreceptor by enhancing the search space, conducting semi-automated high throughput screening of proteins, by ligand binding interactions followed by molecular dynamics simulations for validation and subsequently selecting peptides based on design considerations. The docking module 110A inputs protein structures and target ligand parameters are input into the system 100. The system 100 employs AutoDock Vina^{™}'s docking algorithm to predict potential peptide bioreceptor that may interact with the target ligand i.e. glucose. The output includes ranked protein binding poses, along with predicted binding affinities. The docking results are further validated by molecular dynamics module 110B wherein the predicted binding poses obtained from the high throughput screening are subjected to further validation using molecular dynamics simulations. The simulations consider various environmental factors, such as solvent effects and temperature, to refine the binding predictions. In an embodiment, the memory 102 may include a database or a repository 108. The memory 102 may comprise information pertaining to input(s)/output(s) of each step performed by the processor(s) 104 of the system 100 and methods of the present disclosure. In an embodiment, the database may be external (not shown) to the system 100 and coupled via the I/O interface 106. In an embodiment, the memory 102 includes a database 108. The database comprises of a plurality of sets comprising a set of protein-ligand complexes of various synthetic as well as naturally available proteins like glucose/ galactose binding protein (GGBP), sugar binding proteins, transport proteins, lectins with β-D-glucose. The memory 102 further includes a plurality of modules (not shown here) comprises programs or coded instructions that supplement applications or functions performed by the system 100 for executing different steps involved in the process of identifying nucleomodulins. The plurality of modules, amongst other things, can include routines, programs, objects, components, and data structures, which perform particular tasks or implement particular abstract data types. The plurality of modules may also be used as, signal processor(s), node machine(s), logic circuitries, and/or any other device or component that manipulates signals based on operational instructions. Further, the plurality of modules can be used by hardware, by computer-readable instructions executed by the one or more hardware processors 104, or by a combination thereof. The plurality of modules can include various sub-modules (not shown). Functions of the components of system 100 are explained in conjunction with flow diagrams depicted in FIG. 2 for designing a baseline peptide for sensing a biomarker of dysglycemic disorders.

FIG. 2 illustrates sequential approach adopted by the system 100 in attempting bioreceptor design according to some of the embodiments of the present invention.

As illustrated in FIG. 2, the sequential approach of designing a peptide by the system 100 is presented. The approach comprises automated rapid screening 202 of a plurality of proteins which are considered as potential candidates for peptide selection and systematically designing a peptide bioreceptor using multiple design parameters 210. The automated rapid screening is a three-step procedure involving screening of candidate proteins 204 that have affinity for a ligand of interest (B-D-Glucose) using AutoDock Vina^{™} software suite specialized in high throughput protein-ligand docking tool. The output of AutoDock Vina^{™} ranks the best suitable protein-ligand interactions based on scoring for binding affinity. Next step include umbrella sampling 206 of selected protein-ligand interactions ranked in the previous step using molecular dynamics (MD). The MD is performed using GROMACS^{™} tool and it further validates the results of high throughput screening. The MD factors the effect of environment on the protein-ligand interactions by considering the solvent effect. The interaction is analyzed by calculating potential mean force (PMF) among selected protein and ligand complex. Further, peptide selection 210 is performed by exploring peptide sequence of the protein with most favorable energy profile of the protein towards the ligand. This involves multiparameter optimization. The optimized protein is further utilized as a template to build a bioreceptor by refining its structure through a plurality of parameters such as sequence length, conformational analysis, solubility profile and the cost of synthesis involved. Therefore, systematic approach of attempting the peptide design resulted in a suitable bioreceptor for the ligand of interest (B-D-Glucose).

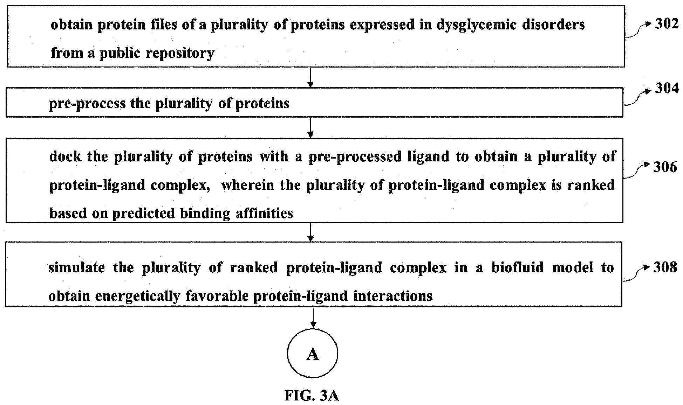

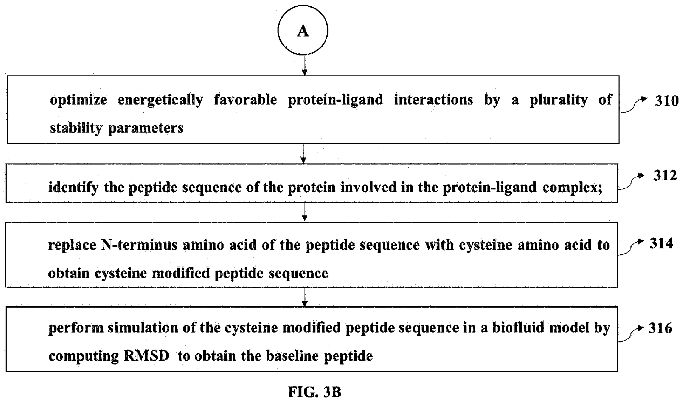

FIGS. 3A and 3B are an exemplary flow diagram for a method for designing a baseline peptide for sensing a biomarker for dysglycemic disorders according to some embodiments of the present disclosure.

In an embodiment, the system 100 comprises one or more data storage devices or the memory 102 operatively coupled to the processor(s) 104 and is configured to store instructions for execution of steps of the method 300 depicted in FIG. 3 by the processor(s) or one or more hardware processors 104. The steps of the method of the present disclosure will now be explained with reference to the components or blocks of the system 100 as depicted in FIG. 1 and the steps of flow diagrams as depicted in FIG. 3. Although process steps, method steps, techniques or the like may be described in a sequential order, such processes, methods, and techniques may be configured to work in alternate orders. In other words, any sequence or order of steps that may be described does not necessarily indicate a requirement that the steps be performed in that order. The steps of processes described herein may be performed in any order practical. Further, some steps may be performed simultaneously. The present disclosure is directed to design a biomimetic peptide capable of interacting with the analyte of interest i.e. glucose. The peptide is designed by a systematic approach of first understanding the bio-physical interactions among naturally derived proteins generally involved in binding to glucose and then designing a synthetic peptide that can offer similar binding affinity to the glucose as that of the natural proteins. Glucose is the molecule of interest, and the disclosed method is directed to design a peptide for binding with glucose. Therefore, the peptide becomes the bioreceptor for glucose.

At step 302 of the method 300, the one or more hardware processors 104 are configured to obtain protein files of a plurality of proteins expressed in dysglycemic disorders from a public repository. The plurality of files of naturally derived protein were imported from protein data bank (PDB), a public repository. The naturally available proteins like glucose/ galactose binding protein (GGBP), sugar binding proteins, transport proteins, lectins, etc were imported. At step 304 of the method 300, the one or more hardware processors 104 are configured to pre-process the plurality of proteins. The pre-processing involves lot many curations to offer a protein before it can be docked. All the non-protein parts are identified and removed from the protein, such as ligands (HETATM), ions (CL, NA etc) and water (SOL). Then, the missing hydrogens are added. Further, appropriate force fields were selected, Kollman charges were added. The pre-processed files are stored internally as PDBQT files compatible with the AutoDock Vina^{™}, a protein docking software. Similarly, ligand is also pre-processed to ensure completeness of the ligand. Generally, the ligand lacks hydrogen atoms, so hydrogen atoms are added to the ligand and rotatable bonds are defined that are used for flexible docking. The pre-processing of proteins and ligands are executed in AutoDock Vina^{™}. At step 306 of the method 300, the one or more hardware processors 104 are configured to dock the plurality of proteins with a ligand to obtain a plurality of protein-ligand complex, wherein the plurality of protein-ligand complex is ranked based on predicted binding affinities. For each protein-ligand complex being assessed by the docking study, the protein is placed in a grid box of known dimensions, the ligand is placed in its vicinity, and the binding pose, and binding energy are obtained by optimizing the potential energy. When a ligand docks into the binding pocket of a protein, it interacts with the neighboring residues by many kinds of non-covalent, non-permanent interactions like hydrophobic interactions, pi-Alkyl interactions, etc. To know if a particular ligand can bind to a protein or to rank two different protein-ligand complexes, the binding energy of the complex is calculated, Typically, docking tools rely on knowledge-based rules learnt from peptides or proteins that bias the conformational search performed to identify the best predicted structure.

### Specific interactions of protein-ligand found suitable for further study:

1HKC: A β-D-Glucose-Hexokinase complex: The crystal structure for Human Hexokinase is obtained from the PDB database, with ID 1HKC. Hexokinases are known to phosphorylate hexoses (6-carbon sugars), forming hexose phosphate. They can transfer an inorganic phosphate group from ATP to a substrate. The complexed structure contains 917 amino acid residues, water molecules, two PO4 2-ion, two K+ ion and one beta-D-glucopyranose molecule bound to the binding site. AutoDock Vina^{™} reports a binding energy of -7.38 kcal/ mol for the β-D-Glucose-Hexokinase complex. It is seen that seven different amino acids interact with the glucose molecule in the binding site via eleven hydrogen bonds. Each of the hydroxyl groups of the glucose molecules interacts with the hexokinase via at least one H-bond. The ring oxygen does not participate in H-bonding. The interacting species and the bond lengths are given in the Table-1 below. Strong H-bonds with a bond length less than 2 Å form between glucose O1 and Glu294, O4 and Asp209, O4 and Asn235 and O6 and Asp209.

**Table-1**

| # | Interacting species | Bond lengths (Å) |
|---|---|---|
| 1 | THR172-O1 | 2.36 |
| 2 | LYS173-O6 | 2.17 |
| 3 | ASN208-O3 | 2.04 |
| 4 | ASN208-O4 | 2.39 |
| 5 | ASP209-O4 | 1.92 |
| 6 | ASP209-O6 | 1.73 |
| 7 | ASN235-O4 | 1.98 |
| 8 | GLU260-O2 | 2.01 |
| 9 | GLU260-O3 | 2.00 |
| 10 | GLU294-O1 | 1.96 |

6BGC: A glucose binding protein, W145A variant of TpMglB-2 (Tp0684) with bound glucose: The crystal structure for the glucose/ galactose binding protein is obtained from the PDB database with ID 6BGC. The 3-D structure contains 387 amino acid residues, water molecules, 2 molecules of 1,2-ethandiol, 2 Ca2+ ions and 2 βD-glucopyranose molecules bound to the binding site. The binding energy of the protein-ligand complex as reported by AutoDock Vina^{™} is -6.19 kcal/mol. Six different amino acids hold the glucose molecule in the binding site via eight Hydrogen bonds. In the complex, the ring oxygen forms a H-bond with Asn19, with a bond length of 1.99 Å. The interacting species and the bond lengths are given in the Table-2 below.

**Table-2**

| # | Interacting species | Bond lengths (Å) |
|---|---|---|
| 1 | Asn19-O5 | 1.99 |
| 2 | Asn19-O6 | 1.94 |
| 3 | Arg20-O6 | 2.19 |
| 4 | Arg95-O2 | 1.84 |
| 5 | Asp-195-O2 | 1.77 |
| 6 | Asp-195-O3 | 1.86 |
| 7 | Asn172-O3 | 2.28 |
| 8 | Asn311-O4 | 1.73 |

1EX1: B-D-Glucan exohydrolase: The Beta-D-glucan exohydrolase belongs to a class of beta-D-glucans which are hydrolysed during wall degradation in germinated grain. The crystal structure is taken from the PDB database with ID 1EX1. The complex is comprised of 605 amino acid residues, one molecule of substituted beta-D-glucopyranose, one molecule of 2-acetamido-2-deoxy-beta-D-glucopyranose and one molecule of alpha-D-glucopyranose. As per AutoDock Vina^{™}, the binding energy of the protein-ligand complex is -6.12 kcal/mol. The complex is stabilized by six H-bonds formed with four residues and two carbon-hydrogen bonds one of which is formed between the residue Gly56 and the O6 atom from the glucose molecule and the other is formed between Asp285 and C1 from the glucose molecule. The strongest H-bond is formed between Glu491 and O1 with a bond length of 1.91 Å. The interacting species and the bond lengths are given in the Table-3 below.

**Table-3**

| # | Interacting species | Bond lengths (Å) |
|---|---|---|
| 1 | Asp95-O3 | 2.19 |
| 2 | Asp95-O4 | 2.08 |
| 3 | Asp95-O5 | 1.92 |
| 4 | His207-O2 | 2.25 |
| 5 | Asp285-O2 | 2.05 |
| 6 | Glu491-O1 | 1.91 |

1HIZ: Xylanase T6 (Xt6) from Bacillus Stearothermophilus: Xylanase belongs to a class of enzymes that break down the polysaccharide xylan into xylose, thus breaking down hemicellulose - a major component of plant cell walls. The 3D structure is obtained from the PDB database with ID 1HIZ. The complex contains 379 amino acid residues, water molecules and one each of alpha-D-glucopyranose, alpha-D-galactopyranose and sulfate ion, SO4 2- . It demonstrates a good binding with glucose as illustrated by a binding energy of -5.92, as per AutoDock Vina^{™}. Eight H-bonds hold the glucose molecule in the binding pocket of the xylanase protein. The ring oxygen O5 participates in H-bond with Arg329 with a bond length of 2.58 Å. The interacting species and the bond lengths are given in the Table-4 below.

**Table-4**

| # | Interacting species | Bond lengths (Å) |
|---|---|---|
| 1 | His237-O4 | 2.35 |
| 2 | Gln239-O4 | 1.79 |
| 3 | Asp268-O6 | 1.88 |
| 4 | Gly273-O2 | 1.96 |
| 5 | Gly273-O3 | 1.93 |
| 6 | Arg329-O1 | 2.63 |
| 7 | Arg329-O5 | 2.58 |
| 8 | Asp358-O1 | 2.36 |

1GWW-Alpha-1,3-galactosyltransferase: It belongs to a family of enzymes, which catalyze retaining glycosyltransfer reactions. The 3D structure is taken from the PDB database with the ID 1GWW. As per AutoDock Vina^{™}, the glucose-protein complex demonstrates a binding energy of -5.43 kcal/mol. The ring oxygen O5 forms H-bond with Glu145 with a bond length of 2.02 Å. The interacting species and the bond lengths are given in the Table-5 below.

**Table-5**

| # | Interacting species | Bond lengths (Å) |
|---|---|---|
| 1 | Ile40-O6 | 2.10 |
| 2 | His142-O1 | 2.46 |
| 3 | Glu145-O1 | 3.06 |
| 4 | Glu145-O3 | 2.02 |
| 5 | Glu145-O4 | 2.12 |
| 6 | Glu145-O5 | 2.02 |
| 7 | Glu145-O6 | 2.61 |
| 8 | Glu146-O1 | 1.92 |
| 9 | Glu146-OH1 | 2.81 |
| 10 | Glu146-O2 | 2.06 |

1HSU: Glycogen phosphorylase: Glycogen phosphorylase is one of the enzymes which catalyzes the rate-limiting step in glycogenolysis in animal cells. The 3D structure is obtained from the PDB database with ID 1H5U. The complex comprises 842 amino acid residues, and one each of 5-chloro-1H-indole-2-Carboxylic Acid [1-(4-flurobenzyl)-2-(4-hydroxypiperidin-1YL)-2- oxoethyl]Amide, pyridoxal-5'-phosphate and alpha-D-glucopyranose. As per AutoDock Vina^{™}, the binding energy of the glucose-protein complex is -4.01 kcal/mol. The complex is stabilized by ten H-bonds formed with six residues and one C-H bond formed between C5 and His377. The interacting species and the bond lengths are given in the Table-6 below.

**Table-6**

| # | Interacting species | Bond lengths (Å) |
|---|---|---|
| 1 | Asn284-O4 | 2.17 |
| 2 | Asn284-O6 | 2.11 |
| 3 | His377-O6 | 2.98 |
| 4 | Asn484-O1 | 2.03 |
| 5 | Asn484-O2 | 2.19 |
| 6 | Glu672-O3 | 1.72 |
| 7 | Glu672-O4 | 2.14 |
| 8 | Ser674-O2 | 2.73 |
| 9 | Gly675-O2 | 1.89 |
| 10 | Gly675-O3 | 2.45 |

3S41: Glucokinase: Glucokinase, which phosphorylates glucose, is known to participate in regulating glucose metabolism in liver and pancreatic cells. The 3D structure is taken from the PDB database with the ID 3S41. The complex contains 469 residues, and one each of S41, alpha-D-glucopyranose and sodium ion, Na+. The binding energy of the glucose-protein complex is -3.96. The glucose molecule is bound to the binding site of the protein via five H-bonds formed with three residues. The ring oxygen does not participate in H-bonding. The interacting species and the bond lengths are given in the Table-7 below.

**Table-7**

| # | Interacting species | Bond lengths (Å) |
|---|---|---|
| 1 | Tyr61-O6 | 1.85 |
| 2 | Arg63-O3 | 2.02 |
| 3 | Arg63-O4 | 2.11 |
| 4 | Val452-O1 | 2.17 |
| 5 | Val452-O2 | 2.10 |

3QVP: Glucose Oxidase: The crystal structure for the Glucose Oxidase at a resolution of 1.2 Å is obtained from the PDB database with ID 3QVP. The 3-D structure contains 583 amino acids, water molecules, one substituted beta-D-glucopyranose, three NAD, and one each of FAD, di(hydroxyethyl)ether, glycerol and chloride ion, Cl-. AutoDock Vina^{™} gives a binding energy of -5.22 kcal/mol for the glucose-glucose oxidase complex. The complex is stabilized by eight H-bonds formed with six residues. The ring oxygen does not participate in H-bonding. The interacting species and the bond lengths are given in the Table-8 below.

**Table-8**

| # | Interacting species | Bond lengths (Å) |
|---|---|---|
| 1 | His158-O2 | 2.86 |
| 2 | His158-O3 | 2.03 |
| 3 | Arg176-O3 | 1.99 |
| 4 | Phe215-O2 | 2.16 |
| 5 | Gln345-O4 | 2.22 |
| 6 | Gln345-O6 | 2.37 |
| 7 | Gly346-O6 | 2.46 |
| 8 | Gln347-O1 | 1.76 |

1GCG: Closed unliganded form of the periplasmic glucose/ galactose receptor: The structure of a closed, unliganded form of periplasmic glucose/ galactose receptor at a resolution of 1.9 Å is taken from the PDB database with ID 1GCG. The 3D structure contains 309 amino acid residues, and one calcium ion, Ca2+ . AutoDock Vina^{™} gives a binding energy of -5.04 kcal/mol for the glucose-protein complex. Ten H-bonds stabilize the glucose-protein complex. The ring oxygen does not participate in H-bonding. The interacting species and the bond lengths are given in the Table-9 below.

**Table-9**

| # | Interacting species | Bond lengths (Å) |
|---|---|---|
| 1 | Asn91-O4 | 2.22 |
| 2 | Asn91-O6 | 2.34 |
| 3 | His152-O4 | 2.86 |
| 4 | His152-O6 | 2.52 |
| 5 | Asp154-O3H | 1.98 |
| 6 | Arg158-O2 | 2.30 |
| 7 | Arg158-O3 | 2.56 |
| 8 | Asn211-O1 | 2.42 |
| 9 | Asp236-O1H | 1.91 |
| 10 | Asp236-O2H | 2.00 |

3KOT: structure of citrobacter freundii effector binding domain containing three amino acid substitutions: The crystal structure of the AmpR Effector is obtained from the PDB database with ID 3KOT. The complex is found to contain 219 amino acid residues and glycerol molecules. AutoDock Vina^{™} gives a binding energy of -3.96 kcal/mol for the protein-ligand complex. The glucose molecule is bound to the binding site via six H-bonds formed with four residues. The ring oxygen does not participate in H-bonding. The interacting species and the bond lengths are given in the Table-10 below.

**Table-10**

| # | Interacting species | Bond lengths (Å) |
|---|---|---|
| 1 | Ala13-O3H | 2.12 |
| 2 | Gln33-O1H | 1.83 |
| 3 | Gln33-O2H | 2.04 |
| 4 | Ile34-O1 | 1.84 |
| 5 | Arg113-O4 | 2.24 |
| 6 | Arg113-O6 | 1.93 |

2XQ2: structure of K294A mutant of ySGLT: The structure of sodium/ glucose co-transporter is obtained from the PDB database with ID 2XQ2. The complex is found to contain a dimer with two monomers each having 593 amino acid residues, and each monomer contains three molecules of Di(Hydroxyethyl)Ether. AutoDock Vina^{™} gives a binding energy of -3.81 kcal/mol for the protein-glucose complex. Five different amino acids, namely Glu429, Leu470, Met473, Ser476, and Met477 interact with the ligand via six Hydrogen bonds. The interacting species and the bond lengths are given in the Table-11 below.

**Table-11**

| # | Interacting species | Bond lengths (Å) |
|---|---|---|
| 1 | Glu429-O6H | 2.59 |
| 2 | Leu470-O3H | 2.28 |
| 3 | Met473-O2H | 2.03 |
| 4 | Ser476-O2 | 2.13 |
| 5 | Met477-O3 | 2.18 |
| 6 | Met477-O4H | 2.04 |

1HSJ: SarR-MBP (maltose binding protein) fusion protein: The SarR-MBP (maltose binding protein) fusion protein is a transcription/ sugar binding protein is a transcription activating protein. The 3D structure obtained from the PDB database with ID 1HSJ contains 487 amino acid residues and alpha-D-glucopyranose molecules. AutoDock Vina^{™} gives a binding energy of -3.8 kcal/mol for the protein-glucose complex. The glucose molecule is held in the binding pocket via five H-bonds with four residues. Interestingly, the ring oxygen interacts with Arg415 via H-bond. The interacting species and the bond lengths are given in the Table-12 below.

**Table-12**

| # | Interacting species | Bond lengths (Å) |
|---|---|---|
| 1 | Gln72-O6H | 1.98 |
| 2 | Arg415-O5 | 3.38 |
| 3 | Gln475-O4 | 2.14 |
| 4 | Glu482-O2H | 1.92 |
| 5 | Glu482-O3H | 2.14 |

From the analysis of data obtained from AutoDock Vina^{™}, it is apparent that H-bonding is the prevalent non-temporary, non-covalent interaction happening between the glucose molecule and proteins. The network of H-bonds formed between the ligand and receptor lends specificity to the glucose sensing by the proteins/ receptors. Each hydroxide group of the glucose molecule participates in at least one H-bond with the surrounding residues. Sometimes one hydroxide group interacts with different residues forming multiple H-bonds. Similarly, one residue may interact with multiple hydroxide groups of the glucose molecule. In order of their binding energies, the proteins are ranked in the below Table 13:

**Table-13**

| # | Protein | Binding Energy (kcal/mol) |
|---|---|---|
| 1 | Human hexokinase, 1HKC | -7.83 |
| 2 | Glucose/galactose binding protein, 6BGC | -6.19 |
| 3 | Beta-D-glucan exohydrolase, 1EX1 | -6.12 |
| 4 | Xylanase, 1HIZ | -5.92 |
| 5 | Alpha-1,3-galactosyltransferase, 1GWW | -5.43 |
| 6 | Glucose oxidase, 3QVP | -5.22 |
| 7 | Periplasmic glucose/ galactose receptor, 1GCG | -5.04 |
| 8 | Glycogen phosphorylase, 1H5U | -4.01 |
| 9 | Glucokinase, 3S41 | -3.96 |

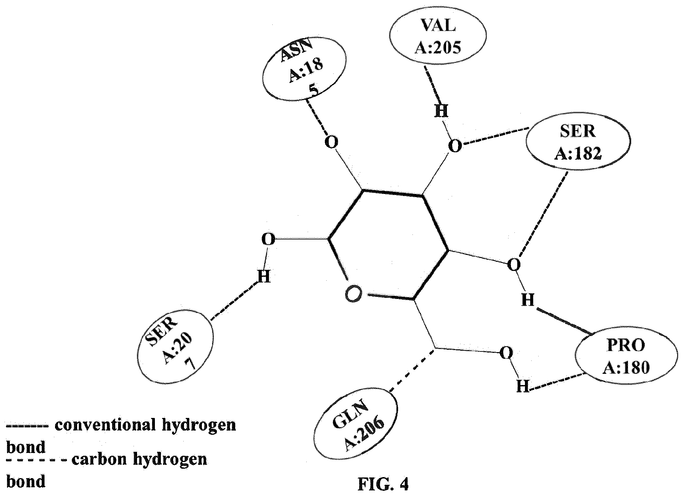

From the above study, interaction of human hexokinase with glucose molecule revealed maximum favorable binding energy as depicted in FIG. 4. As seen in FIG. 4, Aspartic Acid (Asp), Asparagine (Asn) and Glutamic Acid, (Glu) are the most common residues involved in H-bond network. Asp and Glu interact with the hydrogen atom of the hydroxide group via one or both oxygen atoms of their carboxy functional group. Asn interacts with the oxygen atom of the hydroxy group of the glucose molecule via its hydrogen atom of the amide group.

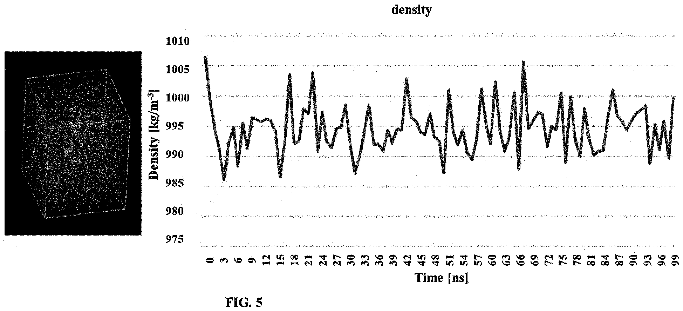

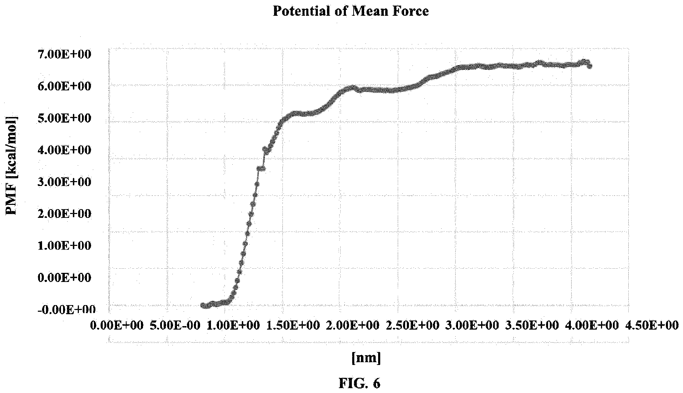

At step 308 of the method 300, the one or more hardware processors 104 are configured to simulate the plurality of ranked protein-ligand complex in a biofluid model to obtain energetically favorable protein-ligand interactions. Based on docking, the most favorable interactions between protein and ligand is identified based on their binding affinity. From the stability and specificity purpose, the spatial arrangement of the residues is performed using molecular dynamics (MD) simulation. The MD simulation performed umbrella sampling using GROMACS^{™} to explore and calculate the free energy profile of a specific reaction coordinate for understanding the energy landscape of the process. Firstly, a reaction coordinate is defined that characterizes the process of interest. i.e., distance between centers of mass of protein and ligand to track interactions and binding events. These are achieved by conducting steered molecular dynamics pull simulation in the desired direction by implementing an imaginary spring. This is termed as constant velocity pull simulation since this rate is fixed for the selected force constant on the spring used for pulling. Subsequently, the potential of mean force (PMF) along this chosen reaction coordinate is calculated. The PMF represents the free energy as a function of the reaction coordinate. Multiple umbrella sampling simulations were performed with the system restrained along the reaction coordinate with each window, a harmonic biasing potential applied to keep the system at a specific value of the reaction coordinate. According to an embodiment, simulation of the cysteine modified peptide sequence involves optimizing (i) binding energies, (ii) conformational changes, and (iii) interactions between the peptides and the target protein. During umbrella sampling, the reactions are carried by considering solvent effect as depicted in FIG. 5. The solvent effect depends on the type of bio-fluid considered for analyzing the analyte (e.g. glucose in this case). When the biofluid is a sweat, then eccrine sweat model is used for MD simulation. When the biofluid is blood, the model depicting blood composition is used. When the biofluid is urine, the model depicting urine composition is used. Subsequently, a histogram of the reaction coordinate values is constructed based on the collected data. This histogram provides information about the probability distribution of the system along the reaction coordinate for reconstructing the PMF. Finally, using statistical mechanics principles, the PMF is reconstructed from the histogram data. This involves applying the WHAM method to obtain the free energy profile as a function of the chosen reaction coordinate. The resulting PMF as depicted in FIG. 6 is utilized in understanding the energetics of the process. It reveals energy barriers, transition states, and stable states along the reaction pathway. Hence, umbrella sampling is employed to quantify the energy landscape and estimate the free energy changes to serve as a validation tool and for estimating the PMF. Pull ligand radially outwards by applying constant force 1000 kJ/mol separating the two and creating configurations for umbrella sampling. Every 0.2 nm frames are selected, and forces computed and, total 40 snap shots and 16 screen shots are selected to study in more depth.

At step 310 of the method 300, the one or more hardware processors 104 are configured to optimize energetically favorable protein-ligand interactions by a plurality of stability parameters. The plurality of parameters comprises selectivity, pH, solubility, sequence length and sequence structure. The effect of all these parameters on the protein-ligand interaction are analyzed individually as well as in combination of more than one parameter at a time to identify deviations in behavior of the interaction.

At step 312 of the method 300, the one or more hardware processors 104 are configured to identify the peptide sequence of the protein involved in the protein-ligand complex. The active binding sites of the top candidate proteins which are found to be most favorable, energetically, were analyzed and candidate continuous sequence of amino acids (< 40 AA) amongst these candidates were iteratively assessed to arrive at a baseline peptide bioreceptor retaining its existing scaffold. Parameters such as binding energy, sequence length, tertiary structure, cost of synthesis, solubility, pH etc. were considered to arrive at the presented peptide. The peptide is modelled in AutoDock Vina^{™} to establish comparable binding energy values and suitable peptide structure. The candidate peptides are optimized by iteratively selecting various continuous lengths from active binding sites of top proteins to arrive at the proposed baseline peptide. Its interference with competing biomolecules such as progesterone, testosterone and glucose are assessed.

At step 314 of the method 300, the one or more hardware processors 104 are configured to replace N-terminus amino acid of the peptide sequence with cysteine amino acid to obtain cysteine modified peptide sequence. The N-terminal is selected as cysteine existing from the native protein which serves as a thiol bond with the gold electrode substrates for further development as a biosensor.

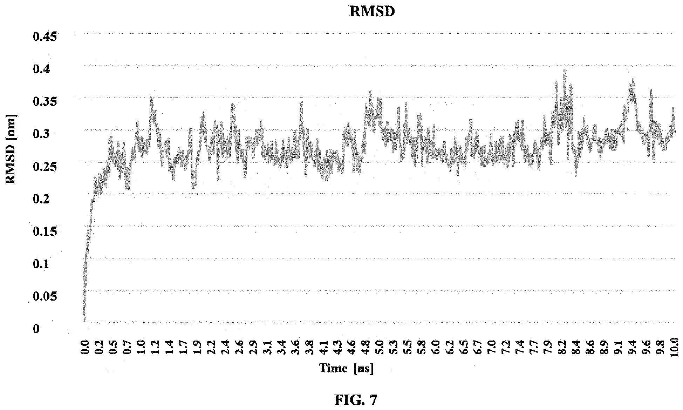

At step 316 of the method 300, the one or more hardware processors 104 are configured to perform simulation of the cysteine modified peptide sequence in a biofluid model by computing root mean square deviation (RMSD) to obtain the baseline peptide. The RMSD between corresponding atoms of two protein chains is a commonly used measure of similarity between two protein structures. To analyze the protein conformations, similarity in three-dimensional structure of native protein and engineered protein is measured by the RMSD of the backbone atomic coordinates after optimal rigid body superposition. The cysteine modified peptide sequence is taken for simulation in a biofluid model by computing RMSD to obtain the baseline peptide. The simulation is performed in GROMACS^{™} for 10ns to ensure stability. The peptide modelled using GROMACS^{™} to assess its root mean square deviation (RMSD) and binding energetics via umbrella sampling and computing PMF and compared with native protein which serves as a benchmark. The approach of designing baseline peptide is biomimetically inspired i.e., selecting a continuous sequence of amino acids from the native protein, correspondingly retaining structure, scaffold and stability of the finalized baseline peptide as opposed to combinatorial peptide design. Any identifiable deviation of structure from the original structure is calculated via RMSD. While performing simulation, individual atom movement is considered. FIG. 7 depicts RMSD variation between 0.2 nanometer (nm) or 2 angstrom (Å). This demonstrate stability of the baseline peptide designed utilizing the method presented in the present disclosure.

According to an embodiment, the biomimetic baseline peptide comprises of amino acid chain that is consciously designed considering physicochemical properties of amino acids, interaction pattern of amino acids with each other and thermodynamically favorable orientations/ conformations along with considering the environmental impact of the type of biofluid. Peptides are sequences of amino acids of varying length and weight. Only 20 of the hundreds of known amino acids account for the vast majority of residues that make up human proteins. The chemical structure of amino acids that occur in protein varies only in the R-group at the carbon in alpha position, Cα, and are referred to as α-amino acids. Not all biologic amino acids are α-amino acids; β-amino acids (e.g., β-taurine), as well as γ-amino acids (e.g., γ-aminobutyric acid), also play important biochemical roles. α-amino acids are chiral, with the exception of glycine, and mainly occur in the 1-form, even though small quantities of D-amino acids occur in biological fluids but without specific function. D-serine is an exception and serves as a neurotransmitter in cerebrospinal fluid. Isoleucine and threonine have a second asymmetric carbon that gives rise to stereoisomers. Furthermore, a number of rare amino acids are recovered from protein hydrolysates, in addition to the 20 protein-forming amino acids. The chemical behavior of the R group dictates the chemical behavior of each α-amino acid, which can be categorized as charged, hydrophilic, or hydrophobic. The hydrophobic amino acids with aliphatic residues are alanine (A), isoleucine (I), leucine (L), methionine (M), and valine (V). Amino acids with aromatic residues such as phenylalanine (F), tryptophan (W), and tyrosine (Y) are also hydrophobic. The aliphatic residues generally provide a hydrophobic environment whereas aromatic residues are usually involved in π-π stacking. The hydrophilic, polar residues participate in hydrogen bonding either via OH (serine (S) and threonine (T)) or CONH₂ groups (asparagine (N) and glutamine (Q)). The ionizable residues can be positively charged, (histidine (H), lysine (K), and arginine (R)) or negatively charged, such as in aspartic acid (D) and glutamic acid (E). Other natural amino acids are cysteine (C), glycine (G), and proline (P). Proline exhibits hydrophobic behavior, whereas cysteine, with its thiol side chain, is an important source of sulfur in human metabolism. Peptides adopt specific conformations depending on the position of each R-group in the amino acid sequence. The secondary structure of peptides (α- helices, β-sheets, and β-hairpins) is driven by noncovalent intermolecular interactions, such as hydrogen bonding, van der Waals forces, π-stacking, and hydrophobic and electrostatic interactions. The secondary structure of peptides can be modulated by introducing modifications in the amino acids, thus favoring interactions with other peptides or proteins. Amino acids can be considered as natural molecular building blocks that spontaneously arrange themselves to form highly organized structures with well-defined functional properties. Recently, peptides have been proposed to be interesting and versatile tools for the design and development of biosensors. Peptides can be easily obtained by chemical synthesis methods, avoiding the need for in-vivo, laborious procedures, as in the case of antibodies. Moreover, they are also biocompatible.

According to an embodiment of the present disclosure, the baseline peptide for sensing a biomarker obtained from the system 100 using the method 300 is represented by a general formula as:

N-terminus-CYS-W-X-C-terminus,

Wherein, X is a sequence of 1-5 charged amino acid;
W is a sequence of between 2 and 70 amino acids of any type, wherein more than 50% of the total amino acids represented by W are non-polar amino acid; and
wherein the N-terminal amino acid is cysteine (CYS) that facilitates binding of the peptide to the gold substrate from N-terminus side utilizing thiol bond of cysteine (CYS) amino acid.

According to an embodiment, the baseline peptide comprises of 2-70 amino acids, preferably 10-50 amino acids, and more preferably 20-40 amino acids. The baseline peptide is designed by keeping amino acids cysteine at the N-terminus, 1-5 charged amino acids at C-terminus, and rest 5-50 amino acids aligned based on sequences derived from docking and simulation with more than 50% amino acids being hydrophobic.

According to an embodiment, the baseline peptide bioreceptor designed according to present disclosure is represented as SEQ ID No 1 has an amino acid sequence as CQLIQMDYVGBGTAFFILPBZGMBTVIALSRDTIDR having a binding affinity as -6.05 kcal/ mol. The physicochemical properties of the peptide sequence ID. No. 1 are disclosed below in Table-14.

**Table-14**

| **Physicochemical properties** | **Value** |
|---|---|
| Number of residues: | 37 |
| Molecular weight: | 4146.69 g/mol |
| Extinction coefficient: | 1280 M⁻¹cm⁻¹ |
| Iso-electric point: | pH 3.53 |
| Net charge at pH 7: | -3.1 |

According to an embodiment, the conformation of baseline peptide was analysed. The baseline peptide demonstrated energetically favorable conformations with a binding energy ranging from about -3.0Kcal/mol to -7.5Kcal/mol of a ligand-biopeptide complex.

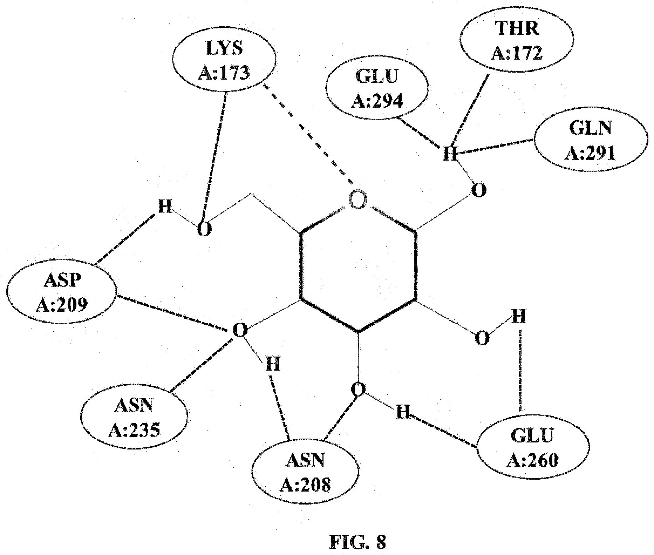

FIG. 8 represents a bioreceptor interacting with β-D-glucose forming a protein-ligand complex with favorable energy profile according to some embodiments of the present disclosure.

As illustrated in FIG. 8, the biopeptide so designed by a method comprising a sequential approach of docking, molecular dynamics and multi-parameter optimization has a cystine amino acid at N-terminus. Further, at C-terminus, biopeptide has a sequence of 1-5 charged amino acid. The biopeptide is 2-50 amino acid long with more than 50% of the total amino acids being non-polar. And lysine at A:173, glutamate at A: 294, threonine at A: 172, glutamine at A: 291, glutamate at A: 260, asparagine at A:208, asparagine at A:235 and aspartic acid at A:209 are connected to β-D-glucose ligand through hydrogen bonding.

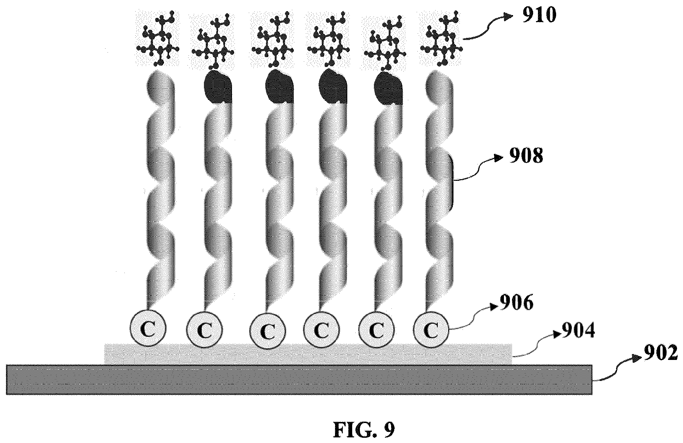

FIG. 9 illustrate a baseline peptide biosensor, according to some embodiments of the present disclosure.

As illustrated in FIG. 9 the biosensor comprises of a baseline peptide bioreceptor offering an electrochemical biosensor that comprises of a substrate 902. A wide variety of materials have been used as substrates for electrodes including paper, polyethylene teraphalate (PET), kapton film, cyclein olefin copolymer, and cellulose, making them favorable for flexible electronics. The substrate layer 904 contacting the baseline peptide is made of gold. Gold electrodes are some of the most prevalent electrochemical biosensor substrate materials because they are readily functionalized with thiolated biomolecules. Yet, conventional methods to fabricate gold electrodes are costly and require onerous equipment, precluding them from implementation in low-resource settings (LRS). Recently, a number of alternative gold electrode fabrication methods have been developed to simplify and lower the cost of manufacturing. These methods include screen and inkjet printing as well as physical fabrication with common materials such as wire or gold leaf. All electrodes generated with these methods have successfully been functionalized with thiolated molecules, demonstrating their suitability for use in biosensors. The Gold is employed as a substrate layer and is readily modified with cysteine 906 which is a thiolated molecules through the spontaneous formation of gold-thiol bonds. The baseline peptide 908 has a cysteine amino acid 906 at N-terminus to form the gold-thiol bonds. The analyte of interest 910 (β-D-glucose in this case) binds to the baseline peptide 908 to enable electrochemical sensing of the analyte of interest. The baseline peptide 908 comprises of more than 50% non-polar amino acid. The biosensor comprises of the baseline peptide 908 as per FIG. 8.

According to an embodiment of the present disclosure, the biosensor further comprises of a transducer, wherein the baseline peptide transduces detectable signal through cysteine amino acid attached to a substrate of the transducer, and wherein a signal transduced by the baseline peptide bound to the biomarker differs from a signal transduced by the baseline peptide when the baseline peptide is not bound to the analyte (biomarker), and wherein the baseline peptide comprises of more than 50% non-polar amino acid.

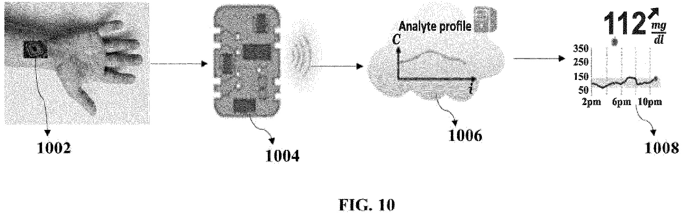

FIG. 10 illustrate a baseline peptide-based device sensing a biomarker in a test sample, according to some embodiments of the present disclosure.

As illustrated in FIG. 10, the biosensor 1002 is in contact with the skin so as to receive the test sample. The biosensor may be in the form of a wearable device which may be worn onto body parts, such as wrist band to be worn on the wrist and the like. The test sample contacts the transduction unit 1004 to perceive the test sample for analysis purpose. Transduction units may be one of electrochemical, optical, thermal or gravimetric sensing techniques. In a preferred embodiment of the present disclosure, the electrochemical based transduction unit is configured to process the test sample. The sample once processed by the transduction unit 1004, signals thus generated are processed at data processing unit 1006. The data processing unit may comprise of one or more amplifiers, processors, signal converters and data communication modules. Based on data processing the outcome of analysis as the concentration of the analyte in the test sample is displayed by a display device 1008. According to an embodiment, the method of detecting the biomarker (or the analyte) in the test sample comprising the steps:
a test sample comprising a biofluid, wherein the biofluid potentially contains a biomarker;
a biosensor comprising a baseline peptide;
contacting the test sample with a surface of the biosensor;
permitting signal generation to occur as the biomarker contacts the baseline peptide of the biosensor; and
detecting the presence or amount of the biomarker in the test sample using a detection assembly.

According to an embodiment, the biosensor is in the form of a wearable device wherein the wearable device comprising a baseline peptide capable of sensing glucose is illustrated. The wearable device comprises of a biosensor array, a power source, a data processor and a data transmitter. The biosensor array comprising a baseline peptide (general formula: N-terminus-CYS-W-X-C-terminus) along a circumference of the wearable device including at least one electromagnetic energy emitter and at least one electromagnetic energy receiver. A power source is provided to the wearable device. A data processing unit receives data from the electromagnetic energy receiver which is analyzed in order to measure glucose level of a subject; and a data transmitter that transmits the signal that are ultimately displayed on a display of the wearable device.

The biosensor does not require a separate thiol linker to bridge the gold plate and the baseline peptide as the N-terminal amino acid, cysteine offers thiol bond to be linked to the gold plate. The device is highly integrated and does not need external auxiliary equipment. The glucose concentration can be obtained through an LCD screen mounted on a wearable device. Through experimental detection, the detection device designed has high detection accuracy. In particular, it can obtain the glucose content in the human body through noninvasive detection, which shows the broad prospect of the application of the detection device in the field of health examination and monitoring.

According to an embodiment of the present disclosure, a process of identifying a biomarker in the biofluid using a baseline peptide is disclosed. The process comprising steps:
a test sample comprising a biofluid, wherein the biofluid potentially contains a biomarker;
a biosensor comprising a baseline peptide;
contacting the test sample with a surface of the biosensor;
permitting signal generation to occur as the biomarker contacts the baseline peptide of the biosensor; and
detecting the presence or amount of the biomarker in the test sample using a detection assembly.

According to an embodiment of the present disclosure, a β-D-glucose, a biomarker for dysglycemic disorders is identified in the plurality of biofluids such as sweat, urine, saliva, blood and the like. According to an embodiment of the present disclosure, a β-D-glucose is identified in the sweat as a non-invasive means of identification and monitoring the dysglycemic disorders. The baseline peptide utilized in biomarker identification is selected from the peptide sequence disclosed as sequence ID No. 1.

The written description describes the subject matter herein to enable any person skilled in the art to make and use the embodiments. The scope of the subject matter embodiments is defined by the claims and may include other modifications that occur to those skilled in the art. Such other modifications are intended to be within the scope of the claims if they have similar elements that do not differ from the literal language of the claims or if they include equivalent elements with insubstantial differences from the literal language of the claims.

The embodiment, thus provide a baseline peptide capable of sensing glucose analyte from the biofluid. The baseline peptide is designed by integrating computational techniques such as AutoDock Vina^{™} and molecular dynamics (MD) simulations that has enhanced the accuracy and efficiency of the method proposed in the present disclosure. The method provides selection of potential candidate for a biopeptide that can sense the glucose analyte in the biofluid. The semi-automated screening via python scripts and AutoDock Vina^{™} and ranking via the scoring function based on binding affinity resulted in a biomimetically inspired baseline peptide. The baseline peptide is modelled using GROMACS^{™} to assess its root mean square deviation (RMSD) and binding energetics via umbrella sampling and computing PMF and compared with native protein which serves as a benchmark. Through modelling, key interactions between target analyte with the candidate bioreceptor are identified. The biosensor device designed utilizing the baseline peptide is sensitive enough to detect glucose in the biofluid, preferably sweat.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g., any kind of computer like a server or a personal computer, or the like, or any combination thereof. The device may also include means which could be e.g., hardware means like e.g., an application-specific integrated circuit (ASIC), a field-programmable gate array (FPGA), or a combination of hardware and software means, e.g., an ASIC and an FPGA, or at least one microprocessor and at least one memory with software processing components located therein. Thus, the means can include both hardware means, and software means. The method embodiments described herein could be implemented in hardware and software. The device may also include software means. Alternatively, the embodiments may be implemented on different hardware devices, e.g., using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items or meant to be limited to only the listed item or items. It must also be noted that as used herein and in the appended claims, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

## Claims

1. A baseline peptide bioreceptor comprising an amino acid sequence of the general formula:
N-terminus-CYS-W-X-C-terminus;
wherein X is a sequence of 1-5 charged amino acid;
W is a sequence of between 2 and 70 amino acids of any type, wherein more than 50% of the total amino acids represented by W, are non-polar amino acid; and
wherein the peptide binds to the gold substrate from N-terminus side utilizing thiol bond of cysteine (CYS) amino acid.

2. The baseline peptide bioreceptor as claimed in claim 1, wherein the ligand binds to the receptor with a binding energy of about -3.0Kcal/mol to -7.5Kcal/mol to form a ligand-receptor complex.

3. The baseline peptide as claimed in claim 1, wherein the peptide sequence is set forth in SEQ ID No 1.

4. A processor implemented method of designing a baseline peptide bioreceptor for sensing a biomarker of dysglycemic disorders, the method comprising steps of:
obtaining (302), via one or more hardware processors, protein files of a plurality of proteins expressed in dysglycemic disorders from a public repository;
pre-processing (304), via the one or more hardware processors, the plurality of proteins;
docking (306), via the one or more hardware processors, the plurality of proteins with a ligand to obtain a plurality of protein-ligand complex, wherein the plurality of protein-ligand complex is ranked based on predicted binding affinities;
simulating (308), via the one or more hardware processors, the plurality of ranked protein-ligand complex in a biofluid model to obtain energetically favorable protein-ligand interactions;
optimizing (310), via the one or more hardware processors, a plurality of stability parameters by docking the stable protein-ligand complex;
identifying (312), via the one or more hardware processors, the peptide sequence of the protein involved in the protein-ligand complex;
replacing (314), via the one or more hardware processors, N-terminus amino acid of the peptide sequence with cysteine amino acid to obtain cysteine modified peptide sequence; and
performing (316), via the one or more hardware processors, simulation of the cysteine modified peptide sequence in a biofluid model by computing root mean square deviation (RMSD) to obtain the baseline peptide bioreceptor.

5. The method as claimed in claim 4, wherein the ligand is β-D-Glucose.

6. The method as claimed in claim 4, wherein the biofluid model is selected from eccrine sweat, saliva, blood and urine.

7. The method as claimed in claim 4, wherein the plurality of stability parameters optimized by docking include selectivity, pH, solubility, peptide sequence length and peptide sequence structure.

8. The method as claimed in claim 4, wherein simulation of the cysteine modified peptide sequence involves optimizing (i) binding energies, (ii) conformational changes, and (iii) interactions between the peptides and the target protein.

9. The method as claimed in claim 4, wherein the baseline peptide bioreceptor detects a biomarker in a test sample by a process comprising steps:
a test sample comprising a biofluid, wherein the biofluid potentially contains a biomarker;
a biosensor comprising a baseline peptide;
contacting the test sample with a surface of the biosensor;
permitting signal generation to occur as the biomarker contacts the baseline peptide of the biosensor; and
detecting the presence or amount of the biomarker in the test sample using a detection assembly.

10. The baseline peptide bioreceptor as claimed in claim 1, wherein the baseline peptide is in the form of a biosensor comprising:
a baseline peptide bioreceptor; and
a transducer, wherein the baseline peptide binds with the biomarker ligand and a detectable signal is transduced through cysteine amino acid attached to a gold electrode of the transducer, and wherein a signal transduced by the baseline peptide bound to the biomarker differs from a signal transduced by the baseline peptide when the baseline peptide is not bound to the biomarker, and wherein the baseline peptide comprises of more than 50% non-polar amino acid.

11. The baseline peptide as claimed in claim 9, wherein the baseline peptide is in the form of a kit comprising:
a biosensor comprising a baseline peptide;
a unit for signal processing; and
a means for wireless transmission.

12. The biosensor as claimed in claim 9, wherein the biosensor is in the form of a wearable device for monitoring of biomarkers of dysglycemic disorders comprising:
a biosensor array comprising the baseline peptide along a circumference of the wearable device including at least one electromagnetic energy emitter and at least one electromagnetic energy receiver;
a power source;
a data processor which receives data from the electromagnetic energy receiver which is analyzed in order to measure glucose level of a subject; and
a data transmitter.

13. A system (100), comprising:
a memory (102) storing instructions;
one or more communication interfaces (106), and
one or more hardware processors (104) coupled to the memory (102) via the one or more communication interfaces (106), wherein the one or more hardware processors (104) are configured by the instructions to:
obtain, protein files of a plurality of proteins expressed in dysglycemic disorders from a public repository;
pre-process, the plurality of proteins;
dock, the plurality of proteins with a ligand to obtain a plurality of protein-ligand complex, wherein the plurality of protein-ligand complex is ranked based on predicted binding affinities;
simulate, the plurality of ranked protein-ligand complex in a biofluid model to obtain energetically favorable protein-ligand interactions;
optimize, a plurality of stability parameters by docking the stable protein-ligand complex;
identify, the peptide sequence of the protein involved in the protein-ligand complex;
replace, N-terminus amino acid of the peptide sequence with cysteine amino acid to obtain cysteine modified peptide sequence; and
perform, simulation of the cysteine modified peptide sequence in a biofluid model by computing root mean square deviation (RMSD) to obtain the baseline peptide bioreceptor.

14. The system as claimed in claim 13, wherein simulation of the cysteine modified peptide sequence involves optimizing (i) binding energies, (ii) conformational changes, and (iii) interactions between the peptides and the target protein.

15. One or more non-transitory machine-readable information storage mediums comprising one or more instructions which when executed by one or more hardware processors cause:
obtaining protein files of a plurality of proteins expressed in dysglycemic disorders from a public repository;
pre-processing the plurality of proteins;
docking the plurality of proteins with a ligand to obtain a plurality of protein-ligand complex, wherein the plurality of protein-ligand complex is ranked based on predicted binding affinities;
simulating the plurality of ranked protein-ligand complex in a biofluid model to obtain energetically favorable protein-ligand interactions;
optimizing a plurality of stability parameters by docking the stable protein-ligand complex;
identifying the peptide sequence of the protein involved in the protein-ligand complex;
replacing N-terminus amino acid of the peptide sequence with cysteine amino acid to obtain cysteine modified peptide sequence; and
performing simulation of the cysteine modified peptide sequence in a biofluid model by computing root mean square deviation (RMSD) to obtain the baseline peptide bioreceptor.
